(19) Europäisches Patentamt / European Patent Office / Office européen des brevets

(11) EP 3 278 735 A1

## (12) EUROPEAN PATENT APPLICATION

(43) Date of publication:
07.02.2018 Bulletin 2018/06

(21) Application number: 16182619.3

(22) Date of filing: 03.08.2016

(51) Int Cl.:
A61B 8/04 (2006.01)       A61B 8/08 (2006.01)
A61B 5/0215 (2006.01)     G01L 11/06 (2006.01)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(71) Applicant: PI-Harvest Holding AG
8200 Schaffhausen (CH)

(72) Inventors:
• BRENNER, Alexander
32688 Haifa (IL)
• BRODSKY, Yuri
3312406 Haifa (IL)

(74) Representative: KIPA AB
P O Box 1065
251 10 Helsingborg (SE)

(54) **A SYSTEM AND METHOD FOR NON-INVASIVE MEASUREMENT OF PRESSURE INSIDE A BODY INCLUDING INTRAVASCULAR BLOOD PRESSURE**

(57)     A device (140) system and method for the non-invasive ultrasound measurement of the intravascular blood pressure is presented, wherein the blood pressure measurements are performed by means of the registration of the distances changes between the surface of the active piezoelectric transducer radiating an ultrasound beam into the localization area of the blood vessel or the heart chamber and the surface elements of the passive ultrasound beam reflector implanted into the blood vessel or the heart chamber. The invention is applicable to any medium transparent for ultrasound waves with the need to measure pressure changes of the liquid.

**Fig. 1**

EP 3 278 735 A1

**Description**

Field of the Invention

The present disclosure pertains to the field of implantable medical devices and systems as well as related methods. More particularly it relates to a system for the non-invasive ultrasound measurement of the intravascular blood pressure. Further, the disclosure relates to ultrasound beam reflectors, ultrasound transducers and receivers and methods for intravascular blood pressure measurement.

Background of the Invention

Diseases including congestive heart failure (CHF), abdominal aortic aneurysm (AAA), pulmonary artery hypertension (PAH), are a major cause of premature death. There is a desire to be able to provide an advantageous monitoring of intravascular and/or intracardial blood pressure, including continuous monitoring. Based on such blood pressure measurements, diagnosis and treatment of patients can be based on a unique level, preventing substantial populations of patients from premature death.

Implantable sensors are that implanted via endovascular techniques. There are passive sensors which are typically electromagnetic, providing an electromagnetic signal when irradiated from the external to the human body source of electromagnetic energy mainly in radio frequencies (RF). These sensors have electronics incorporated and have thus related disadvantages, like larger size or reliability issues when implanted over time. Moreover, a part of the RF energy is absorbed by an oscillating circuit of the implanted sensor and a part - by the body, which in fact causes potential problems to the living organisms. Energy transmitted from outside the body may be converted in these implants to power the electronics, make measurements and transmit measurement results to the outside of the body again. A detecting system external to the human body registers the electromagnetic field irradiated in its turn by the circuit of the implanted sensor is detected by the detecting system. An example of electromagnetic sensors is described in the United States patent number US 7245117 B1 with the title "Communicating with implanted wireless sensor", the resonant frequency of a sensor is determined for energizing the system to burst the RF energy at predetermined frequencies and amplitudes. A similar technology is described in the United States patent number US 8894582 B2.

Intravascular ultrasound measurements are known, but restricted to either catheter based ultrasound transceivers introduced into the body, mainly for imaging and Doppler ultrasound measurements. Blood pressure in peripheral vessels may be measured from externally the body based on ultrasound, but has issues with calibration and cannot measure selectively at specific deeper places in the body, like e.g. the aorta or the heart. Non-invasive techniques include blood vessels dimensions examination, and the blood flow estimation which are based on the Doppler ultrasound and imaging ultrasound methods, such as for instance disclosed in US 5411028, US 5477858 A, US 5544656, US 6814702 B2, US 5724973 A, US 20140081144 A1, EP 1421905 A1, US 7128713 B2, WO 2007087522 A2, US 20080119741 A1, US 7736314 B2, US 20130197367 A1, or US 20130006112.

For example in the patent US 5520185 A with the title "Method for recognition and reduction of blood speckle in blood vessel imaging system", a method for enhancing an intravascular ultrasound blood vessel image system is disclosed. It is explained how ultrasound echoes representing vessel walls are distinguished from ultrasound echoes from blood flow by using a classifier which employs the mean and variance of the raw data of grey scale intensities as acquired directly from an ultrasound scanner- detector.

In the patent US 5800356 with the title "Ultrasonic diagnostic imaging system with Doppler assisted tracking of tissue motion", a method for tracing the border of tissue through temporarily acquired scan lines using velocity information corresponding to the tissue edges to trace the denoted border is disclosed.

In the patent US6258031 B1 with the title "Ultrasound diagnostic apparatus", the velocity of a blood flow and velocity of a blood walls at the same time are measured by the ultrasound with phase detecting.

In the patent US 20090171205 A1 with the title "Method and system for locating blood vessels", a method utilizing the direct ultrasound sounding for detecting the blood vessels and precisely determining of their depth and diameter is disclosed.

In the patent US 8469887 B2 with the title "Method and apparatus for flow parameter imaging", a method using pulse-wave spectral Doppler imaging allowed to obtain an ultrasound image as a sectional image of the blood vessel, including the inner and outer walls.

Methods and systems for the blood pressure measurements in the blood vessels using an ultrasound Doppler method include :

US 5749364 A1, WO 20010000 A9, US 20070016037 A1, US 20050015009 A1, US 20140180114 A1, US 20140148702, US 8968203 B2, US 20150289836.

In the published patent application US 20150230774 A1 with the title "Blood pressure monitor and method", non-invasive continuous real-time monitoring of an arterial blood pressure is disclosed using Doppler probes for systolic and diastolic blood pressure.

The above discussed non-invasive ultrasound methods for the examination of the blood vessels using Doppler and imaging ultrasound have a number of explicit deficiencies, which are singly or in combinations desired to be overcome (but not necessarily limited to the below only):

1. Reproducibility and accuracy of the examination of the blood vessel is highly dependent on the correct orientation of the ultrasound beam propagation direction axis (the axis of the ultrasound transducer) relatively to the vessel longitudinal axis under the test. Measuring the speed parameters of the blood flow occurs in the result of converting of the value of the shift of the Doppler frequency $\Delta f$ with use the Doppler equation:

$$V = (c \times \Delta f) / (2f_0 \times \cos \alpha),$$

where: $V$ is the velocity of the blood flow, c is the speed of sound in the tissue, $f_0$ is the initial frequency of the signal, $\alpha$ is the angle between the direction of the blood flow and the axis of the ultrasound beam. The angle $\alpha$ strongly affects the value of the measured Doppler frequency $\Delta f$ which in turn is used to calculate of the speed of the organic reflectors in the blood flow.

2. Reliability and precision of blood vessel examination including blood pressure measurement based on ultrasound can be improved. For instance, the Doppler's frequency spectrum displays the blood flow information from a certain area at a given depth called the control volume and does not provide the information about bloodstream in other parts of the vessel which are visible on the ultrasound image. Therefore, in case of the wrong choice of the control volume (ex., when $\cos \alpha \sim 0$) all diagnostic information will be incorrect.

Moreover, blood vessels examination using ultrasound imaging methods are connected with common drawbacks of the ultrasound diagnostic technology, such as echogenicity of objects under examination and resolution capabilities.

Insufficient accuracy of the examination results of the hemodynamics in blood vessels using certain Doppler methods are for instance observed in S.B.Coffi, D.Th.Ubbink and D.A.Legemate. Non invasive Techniques to Detect Subcritical Iliac Artery Stenosis. Eur.J.Vascular and Endovascular Surgery, 29, 2005; Ricardo Cesar, Rocha Moreira. Comparative study of Doppler ultrasonography with arteriography in the evaluation of aortoiliac occlusive disease. Journal Vascular Brasileiro,8, Jan./Mar. 2009; or Vilhelm Schaberle. Ultrasonography in Vascular Diagnosis.A Therapy-Oriented Textbook and Atlas. Second Edition. Springer Heidelberg-Dordrecht- London-New-York, 2011..
The article Gernot Schulte-Altedorneburg, Dirk W. Droste, Szabolcs Felszegny, Monica Kellerman et al., Accuracy in vivo Carotid B-mode Ultrasound Compared with Patological Analysis: Intimamedia Thickening , Lumen Diameter and Cross-Sectional Area. Stroke: Journal of the American Heart Association, 2001 demonstrates an insufficient accuracy of the results obtained for the examination of blood vessels using of the ultrasound B-mode imaging only. There exist previous patents using passive sensors placed in the human body and interacting with an external ultrasound source for analysis of physiological parameters of the human organism, as for instance US 5619997 A, US 5989190 A, US 6083165 A, or US 20030176789 A1. However, these devices and methods have a number of drawbacks, namely the following:

1. The disclosures in patents US 5619997 A, US5989190 A, US 6083165 A consist in the suggestion that the physical parameters (pressure, temperature, viscosity) defining the state of the medium (including the human body) are determined as a functional relationship $P = f(v)$, where $P$ is the physical parameter and $v$ is the frequency of the ultrasound wave reflected by a passive sensor placed in the medium which is different from the frequency of the primary ultrasound beam due the energy absorption by the sensor.

2. The disclosure in patent application US 20030176789 A1 suggests that the value of a specific physical parameter, as the pressure, associated with the specific state of any medium (including the human body) is determined as the result of the frequency analysis of the acoustic signal reflected by the passive sensor implanted into the medium. The passive sensor has to be equipped with two parallel to each other reflective surfaces and the reflected signal is the result of the interference of the two acoustic signals: the first signal is reflected by the first reflective surface and second signal reflected by the second reflective surface.

The frequency analysis of the resultant signal permits allocate the frequencies of the maximal attenuation of the intensity and the value of the specific physical parameter is determined on the basis of the correlation relationships between the values of the parameters and the frequencies of the maximum attenuation of the resultant signal. The knowledge of the correlation relationships between the values of the parameters and the frequencies is not sufficient to determine the functional relationship $P = F(v)$. The method is dependent of the frequencies of both the direct and reflected signals, It is desired to provide a more simple method and system that is independent of the frequencies of both the direct and reflected signals,

Summary of the Invention

The present invention is defined by the enclosed patent claims. The present disclosure may include basis for further separate or overlapping inventions.

The present invention permits to directly measure pressure and its dynamic changes inside a body from the outside of the body without the need of an actively driven implanted device. For instance the blood pressure and its dynamic changes within the cardiovascular system, including in a blood vessel or intracardially such as in a heart chamber, atrium, appendage etc. is facilitated by means of an implanted passive sensor. The sensor is implanted into the cardiovascular system, such as into an artery or the heart itself. The sensor is preferably implanted minimally invasive via a catheter based technique. Further, the passive sensor has ultrasonic beam reflectors which reflect ultrasound waves generated by ultrasound transducers to be captured by ultrasound receivers.

The disclosure provides for pressure measurement inside the body, such as inside the cardiovascular system is measured by direct ultrasound measurements of the displacements of a movably reflective surface portion of a passive reflector implanted inside the body including the cardiovascular system, such as a blood vessel. $P = F(\Delta L)$ where P is the pressure and $\Delta L$ is approximately the distance between fixed and moving portions of the passive reflector surface. A method is disclosed herein, which is independent of the frequencies of both the direct and reflected signals. Thus prior technical solutions, such as disclosed in patents US 5619997 A, US 5989190 A, US 6083165 A, or US 20030176789 A1 are not analogues both in the methods of data collection and the methods of data processing of the current disclosure.

High accuracy and stability of the ultrasound measurements of the pressure are provided. The blood pressure and its dynamic changes within the blood vessel can be provided with high accuracy and stability by creating a system with the implantable sensor placed inside the blood vessel.

The sensor is highly sensitive to the changes of the pressure, and interacts as a dynamic reflector of the direct sounding ultrasound beam with the modulation of the intensity of the reflected beam. The essence of the current disclosure lies in the development of a direct method of ultrasonic measurement of the blood pressure in the heart or a blood vessel and an apparatus for its practical implementation.

The implanted sensor is highly sensitive to the pressure changes, and interacts as a dynamic reflector of the direct sounding ultrasound beam with the modulation of the intensity of the reflected beam.

The presented method for intravascular blood pressure measurement is based on the presence of two different kinds of ultrasound beam reflecting surfaces:

a) a first fixed surface or surface portion at a constant position, which surface is independent from the intravascular blood pressure changes, the surface being fixed on, onto, to, or in relation to a cardiovascular wall, such as an arterial or heart chamber wall;
b) a second moving surface or surface portion configured to oscillate under pressure changes of a fluid, preferably a liquid such as blood, in fluid communication with the second moving surface or surface portion, wherein the pressure changes preferably are blood pressure changes inside the cardiovascular system, such as inside a blood vessel or the heart.

A system is further provided for subsequent measurements and calculations of local blood pressure values $P_i$ at time moments $t_i$ as the function $P_i = F(\Delta L_i)$ of the difference $\Delta L_i$ between the distances of the first fixed and second moving surfaces to the radiative surface of the ultrasound transducer denoted as $\Delta L = L_1 - h_2$, where $L_1$ is the distance between the radiative surface of the ultrasound transducer and the first fixed surface of the passive reflector and $L_2$ is the distance between the radiative surface of the ultrasound transducer and the second moving surface of the passive reflector respectively, at the measurement time moment $t_i$. The calculation is based on the utilization of the inverse calibration function $\Delta L_i = F^{-1}(P_i)$ which is derived from previous calibration measurements of the dependency $\Delta L_i$ from $P_i$ at varying pressure values in a predetermined operating range of the passive reflector. As the reflectors allow for calibrated measurements, absolute pressure measurements are provided, not only dynamic changes of pressures.

Based on the above principle the system for the non-invasive ultrasound measurement of the intravascular blood pressure comprises a plurality of passive ultrasound beam reflectors implantable into the cardiovascular system, such as blood vessels or one or both of the heart chambers. Said passive reflectors contain surface elements or surface portions that are stationary respectively moving under blood pressure changes and adapted to receive and reflect ultrasound beams.

The ultrasound beam reflectors may be integrated or attached to a medical implantable device adapted for delivery and implantation in the body. Such medical implants include for instance stents including self-expandable stents, or occluders such as atrial septal occluders, ventricular septal occluders, (left) atrial appendage occluders, PDA occluders, vascular occluders, vascular plugs, flow regulators, Atrial Flow Regulators (AFR), Aorto-Pulmonary Flow Regulators (APFR), etc.

The system further may comprise an ultrasound apparatus adapted to send ultrasound signals to the implanted ultrasound beam reflectors and to receive reflected signals in return, for performing the intravascular pressure measurements. The system comprises one or more of the following units:

a) at least one ultrasound probe with at least one transducer configured to convert an electro-magnetic input or control signal into a mechanical ultrasound signal to be transmitted towards a surface, and configured for the reverse conversion of reflected or echoed, incoming mechanical ultrasound signals into electro-magnetic measurement signals, wherein said transducer transmits the direct output ultrasound signal and receives the reflected ultrasound signals;

b) at least one beam former unit configured to provide a desired shape of the electro-magnetic signal in the transmission mode;

c) at least one transmitter unit generating the electro-magnetic signals with their further transformation into ultrasound signals by the transducer;

d) at least one receiver unit for the echoed signals;

e) at least one receiver unit for the information of the signals processing;

f) at least one unit for information data storage; and

g) at least one control unit.

The unit for the information processing includes preferably a software, and is for instance comprised of:

a. an ultrasound apparatus, such as disclosed above, and preferably with a communication interface such as a wireless communication unit and/or USB port capability;

b. a client device such as a smartphone/tablet/personal computer with a user interface and a client application installed; optionally integral part of the ultrasound apparatus (a) and/or in communication thereto;

c. optionally a local medical centre data server; and

d. optionally a cloud information storage unit.

The software system includes code segments and the system is in use operating as follows:

e. Connect the ultrasound apparatus (a) to a client device (b) via suitable communication interface, such as WiFi/Bluetooth/USB, cable;

f. Set the transducer into operation; the user interface, such as a graphical user interface (GUI) including an on-screen image is displayed on a display, preferably of the ultrasound apparatus or a display connected thereto, such as the client display. The ultrasound apparatus is in a first operation mode run in B-mode. For instance the client (b) starts the apparatus to operate in B-mode. A picture formed by the signal is displayed.

g. The transducer is pointed to the region where the reflector for pressure measurement is located inside the body, such as the heart, and hold. Optionally the signal direction is adjusted according to the displayed image until the reflective implanted membrane is visible on the image.

h. When the membrane is identified in the image data, the ultrasound apparatus is switched to a second mode of operation, the M-mode. For instance, the client application (b) provides for the identification. The membrane may be automatically recognized by suitable image processing steps, and switched to M-mode. The transducer is then retrieving the signal changes for a certain time length, preferably for a number of seconds. Pressure inside the body is then calculated by the control unit based on the retrieved reflected signal changes.

i. Upon the successful retrieval, which can be confirmed by suitable software steps in a control unit, the results may be displayed and/or further processed. This may be done by the client application (b). In addition, the ultrasound apparatus may be returned to first operational mode, the B-mode.

j. The measurement results including intra-body pressure values may then be manually or automatically uploaded to a local medical centre server (c), a cloud information storage (d), and/or stored otherwise, e.g. in a memory of the client device.

That is an approach based on a combination of both B-mode and M-mode imaging.

B-Mode or 2D mode or brightness mode in ultrasound scanning, is a cross-sectional image representing body components through the simultaneous scanning by a linear array of transducers and represented as a two-dimensional image on a screen.

M-mode or TM-mode is or motion mode in ultrasound scanning, permits to fix a specific scan line in B-Mode and simultaneously generate its real time evolution as a vector time series.

D-mode or Doppler mode in ultrasound scanning makes use of the Doppler effect in measuring and visualizing blood flow or tissue movements in a given sample volume.

An example of an implantation medical procedure for deployment of the passive ultrasound beam reflector inside the body such as inside the cardiovascular system e.g. an appropriate heart region, left and/or right atrium of the heart, or pulmonary artery, comprises

(a) deployment of a carrier unit including a passive ultrasound beam reflector, inside a catheter sheath, such as being releasably attached, preferably at a proximal end, to a connector, such as a capturing unit, such as a claw, being arranged at a distal end of a delivery unit, such as a guide wire or pusher wire of interventional cardiology, configured for releasable attaching said passive ultrasound beam reflector to said connector;

(b) transvascular transportation of the carrier unit to a cardiovascular location, such as a heart region, through the inside of said sheath by means of guide wire or pusher wire manipulations, e.g. by pushing the carrier unit including the passive ultrasound beam reflector through the sheath towards its distally open end;

(c) orientation and deployment of the said carrier unit by means of said guide wire or pusher wire manipulations at said cardiovascular location, such as inside the heart, preferably guided based on fiducial markers on the capturing unit and/or said delivery unit, such as being visible on ultra-sound or fluoroscopy equipment;

(d) anchoring of the said carrier unit and/or passive ultrasound beam reflector into tissue at said cardiovascular location, such as the heart muscle tissue or a blood vessel wall tissue by means of a tissue anchoring unit, such as at least one screw, hook, spring, flange, or the like, at the carrier unit or passive ultrasound beam reflector, or alternatively or in addition based on shape memory material and characteristics thereof to allow fixation;

(e) releasing said carrier unit from the said capturing unit of the delivery unit;

(f) extracting of the sheath from the heart region and the body.

In particular, the example may include a medical procedure for deployment said passive ultrasound beam reflector of Claim 1 inside the cardiovascular system, said procedure comprising

(a) deploying 810 of said passive ultrasound beam reflector inside a sheath being attached by means of a proximal end 220, 320, 420 to a capturing unit, being arranged at a distal end of a delivery unit for releasable attaching of said passive ultrasound beam reflector to said capturing unit;

(b) endovascular transporting 820 of said carrier unit to an appropriate heart region inside said sheath by means of guide wire manipulations;

(c) orientating 830 of said carrier unit by means of said guide wire manipulations inside the cardiovascular system according to fiducial marks on said capturing unit and said delivery unit, such as visible on ultra-sound or fluoroscopy equipment

(d) anchoring 840 of said passive ultrasound beam reflector;

(e) releasing 850 said carrier unit from said capturing unit of said delivery unit; and

(f) extracting 860 of said sheath from the heart and said body.

The carrier unit may be a medical implantable device as mentioned above.
Releasable attachment may be made in any suitable form, such as threaded screw attachment, gripper, forceps, thermal release attachment, etc.

The present invention permits to directly measure the blood pressure through the passive sensor implanted into an artery or heart itself.

Brief Description of the Drawings

These and other aspects, features and advantages of which embodiments of the invention are capable of, will be apparent and elucidated from the following description of embodiments of the present invention, reference being made to the accompanying drawings, in which

Fig.1 depicts a schematic illustration of a standalone passive ultrasound beam reflector with the ultrasound beam reflecting surfaces deployed in a blood vessel;

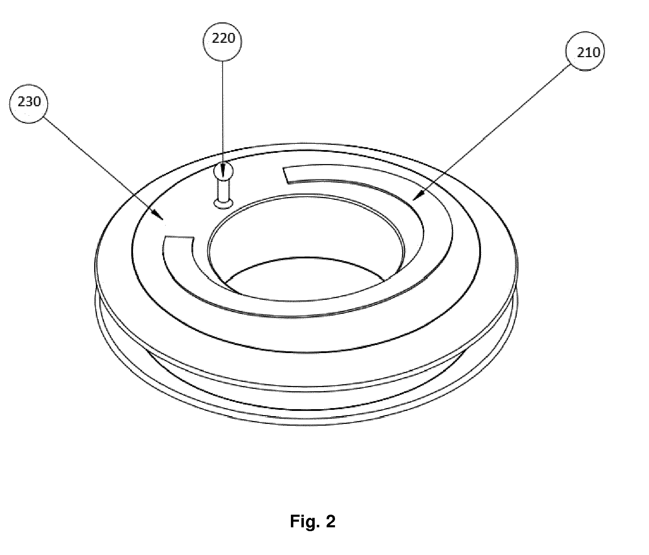

Fig.2 depicts a schematic illustration of a medical implantable device, in an example of an Atrial Flow Regulator (AFR) or APFR (Aorto-Pulmonary Flow Regulator), see Patent Application WO 2016 038115, which is incorporated herein by reference in its entirety for all purposes, namely a passive ultrasound beam reflector, here in form of a membrane with the ultrasound beam reflecting surfaces based measuring the blood pressure in the Right Atrium (the capturing unit resides in the right blood circle); APFR does not significantly differ from AFR device in its geometry, though differs significantly in the method of implantation see Guo K, Langleben D, Afilalo J, Shimony A, Leask R, Marelli A, Martucci G, Therrien J. Anatomical considerations for the development of a new transcatheter aortopulmonary shunt device in patients with severe pulmonary arterial hypertension. Pulm Circ. 2013 Sep;3(3):639-46. doi: 10.1086/674328. Epub 2013 Nov 18, which is incorporated herein by reference in its entirety for all purposes,;

Fig.3 depicts a schematic illustration of the Atrial Flow Regulator (AFR) or APFR (Aorto-Pulmonary Flow Regulator) based passive ultrasound beam reflector with the ultrasound beam reflecting surfaces, deployed in the Left Atrium (the capturing unit resides in the right blood circle);

Fig.4 depicts a schematic illustration of an Atrial Flow Regulator (AFR) based passive ultrasound beam reflector with the ultrasound beam reflecting surfaces deployed both in the Left and Right Atrium (the capturing unit resides in the right blood circle);

Fig.5 depicts a schematic illustration of a blood pressure measuring, registration and reporting medical information system;

Fig.6 depicts a schematic illustration of Ultrasound Transducer interaction with the passive membrane inside the blood vessel or the heart;

Fig.7 depicts a schematic illustration of another 3d visible via Ultrasound Transducer embodiment of both the standalone passive ultrasound beam reflector being attached to a Atrial Flow Regulator (AFR) or APFR (Aorto-Pulmonary Flow Regulator);

Fig.8 depicts a flow chart illustrating an implantation medical procedure; and

Fig.9 depicts a schematic illustration of a passive ultrasound beam reflector being attached as positioned at a distal end of a self-expandable stent, incorporated into an Atrial Flow Regulator (AFR) or APFR (Aorto-Pulmonary Flow Regulator). The reflector is attached to the stent with the help of additional ball acting as immobilizer of the stent relatively to AFR/APFR.

Description of Embodiments

Specific embodiments of the invention will now be described with reference to the accompanying drawings. This invention may, however, be embodied in many different forms and should not be construed as limited to the embodiments demonstrated herein; rather, these embodiments are provided so that this disclosure will be thorough and complete, and will fully convey the scope of the invention to those skilled in the art. The terminology used in the detailed description of the embodiments illustrated in the accompanying drawings is not intended to be limiting of the invention. In the drawings, like numbers may refer to like elements.

The present description of the current invention is given with reference to a blood vessel or a heart chamber as an example only. It should be born in mind however that the present invention is not limited strictly to a blood vessel or a heart chamber, but can be easily adapted to any medium transparent for ultrasound waves with the need to measure pressure changes of the liquid flow. Examples include one or more passive ultrasound beam reflectors positioned in the lymphatic system, bile ducts, urinary ducts, subarachnoid space around the brain and spinal cord (Cerebrospinal fluid), inside or exterior of the lung in the chest wall, etc. for measuring pressures and dynamic progress thereof. Correspondingly, implants adapted for implantation in such a location in the body having at least one attached passive ultrasound beam reflectors are provided.

In accordance with preferred embodiments the system comprises:

   1. One or more passive ultrasound beam reflectors implantable or implanted into the body, such as into the cardiovascular system of a mammal, such as in one or more blood vessels or heart chambers. The reflectors comprise two kinds of surface elements arranged relative each other. Firstly, a stationary (substantially not moving under blood pressure, i.e. a reference reflector surface) surface element adapted to receive and reflect ultrasound beams. Secondly a surface element configured to move under (blood when implanted in the cardiovascular system) pressure changes and adapted to receive and reflect ultrasound beams. The ultrasound beam reflectors hence include a first fixed surface at a constant position independent from the (intravascular blood) pressure changes at its implantation location. Further, a second moving surface is included in the ultrasound reflector, the second surface adapted to oscillate under (blood) or in line with (blood) pressure changes at the implantation site, such as inside a blood vessel or a location of the heart. Preferably, the passive ultrasound beam reflector(s) are provided arranged at a medical implant to be implanted at a location inside the body where pressure changes may occur, as for instance described above.

Preferably, the passive ultrasound beam reflector (or the medical implant with attached passive ultrasound beam reflector(s)) is at least partly endothelialized (overgrown with tissue) after a certain implantation time. In this case, a thin tissue layer will not hinder the movable membrane from oscillation with the pressure of the adjacent fluid (blood) across the tissue layer.

Alternatively, or in addition the surfaces in blood contact can be suitable coated (e.g. heparin coated or with another pharmaceutical substance as desired) and/or being made of a material compatible with blood.

The passive ultrasound beam reflectors beams have suitable shape and sizes for being collapsible into a catheter for delivery, for being attachable to a carrier, and/or for fitting with reliable anchoring at the implantation size. Sizes can be as small as a few mm. The beams can be foldable and be of a resilient material returning to their expanded, substantially planar relaxed shape as shown in the Figs. Shapes include rectangular, square, circular, semi-circular, oval, open oval, generally elongate, or the like.

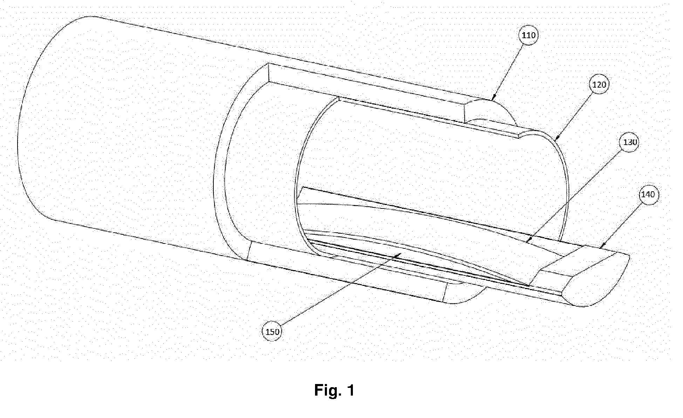

Some exemplary embodiments will now be described. It should be noted that the exemplary embodiments should not be taken as isolated from each other, but features of embodiments may be present in other embodiments, even if not explicitly described or shown in the Figs. For instance, the open ring shaped reflector of Fig. 2 could be in the form of a standalone reflector device, or attached to other medical implants than an AFR. Moreover, rectangular membranes as shown in Fig. 1 could be attached to medical implants, like the AFR in Fig. 2.

   a) In accordance with a first embodiment, the passive ultrasound beam reflector 150 (Fig. 1) is in the form of a plate or contoured membrane which is deformed under the (blood) pressure changes at its location. The device 150 is shown deployed inside a blood vessel, such as the pulmonary artery. In the example, the device is a stand-alone device, i.e. not attached to a carrier medical implant. In similar embodiments, it may be attached to a medical implant carrier, such as a stent. The reflector 150 includes both stationary 140 and movable 130 surface elements, wherein the movable surface element 130 is moving under (blood) pressure changes when implanted. The moveable surface element is attached at its beam ends to a carrier and/or the stationary surface element 140. Thus an oscillatable beam with a free apex is provided that is deflectable by the pressure at its implantation location. The reflector is shown inside the delivery sheath 120 inside the vessel, here the pulmonary artery 110 during transvascular deployment and before release and anchoring in the vessel. Anchoring of the device 150 may be done in many suitable anchoring means implementing ways, such as with hooks, bows, screws, tissue adhesives, etc. The apex is preferably also present in other embodiments described herein. The stationary surface element may be arranged in parallel, above or below the oscillatable beam for reflection in substantially the same direction as the oscillatable beam. Alternatively, or in addition, such a stationary surface may be provided adjacent the oscillatable beam as e.g. shown in Fig. 1 (such as the elevated heel shown at an end of the reflecting device).

b) In accordance with another embodiment, the passive ultrasound beam reflector 210 (Fig.2) in the form of a plate or contour membrane has an open ring shape. An apex as in the previous embodiment may be provided. As mentioned afore, a moveable membrane 210 is deformed under the blood pressure changes. The reflector 210 is attached to an AFR device playing itself the role of the first fixed surface 230 and having two flanges to be positioned on two different sides of the heart across a septal shunt. The AFR device allows a blood flow through its central passageway as shown. The flanges (here disk formed) retain AFR device at the septal wall in its position. It usually is overgrown with endothelia a certain time after implantation. The moveable reflector part is attached to the proximal end on the surface of the AFR where in the example a delivery connector or capturing unit 220 is positioned on the AFR (Atrial Flow Regulator) device 230.when the AFR is implanted, the aggregate of AFR and reflector 210 allows for a controlled shunt between left and right atria. The reflector 210 provides for pressure measurement on the proximal side of the AFR. Being able to measure pressure at an AFR is a long felt need and provided in an advantageous way by these embodiments having an integrated pressure measuring reflector 210 with an AFR. Pressure is an important parameter to determine the effective implantation of an AFR as the shunt is created to treat a hypertonic condition by providing a desired shunt flow between the right and left heart. Alternatively, or in addition the reflector 210 may be attached to or integrated with other medical implants than AFR devices, such as medical implants of the occlusion devices type including Atrial Septal Occluders, Ventucular Septal Occluders, stents, etc.

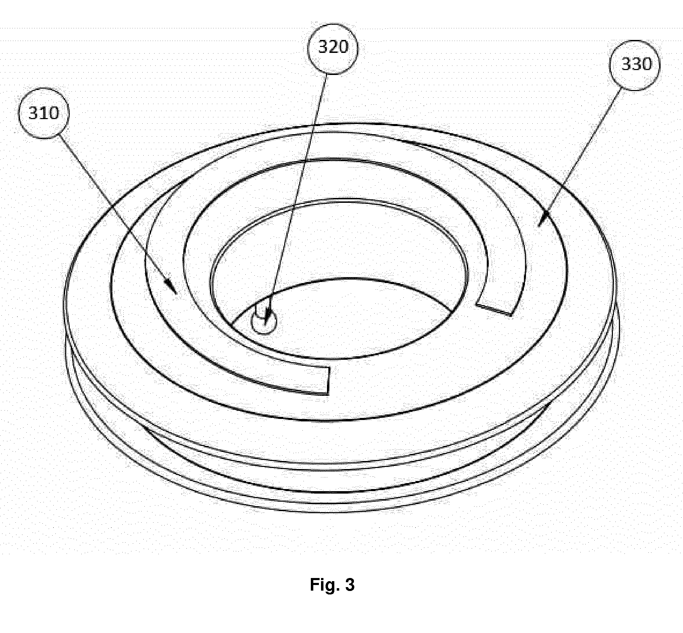

c) In accordance with another embodiment, the passive ultrasound beam reflector 310 (Fig.3) is provided in the form of a plate or contoured membrane in the form of an open ring. The reflector 310 is deformed under the blood pressure changes and is attached to the distal end surface of an AFR, i.e. on the surface opposite to the delivery connector or capturing unit 320 of the AFR (Atrial Flow Regulator) device 330, when deployed to create a shunt between left and right ventricles as described above.

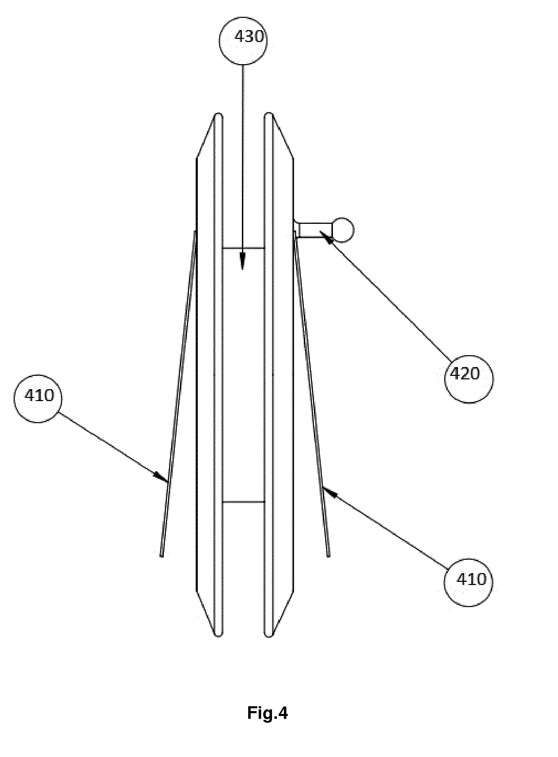

d) In accordance with another embodiment, two passive ultrasound beam reflectors 410 (Fig.4) in the form of a plate or contoured membrane deformed under the blood pressure changes are attached to both the distal and proximal end of an AFR (Atrial Flow Regulator) device 430 deployed to create a shunt between left and right ventricles. It can be seen that the reflectors 410 moveable surface portion, as shown in Fig. 2 or 3, of open ring shape protrude at their apex from the AFR device and are attached at their ring ends only to provide an increased oscillation. The illustrated dimension of the protrusion may be smaller in order to not hinder endothelisation. A ring may be closed as an alternative and attached at a perimeter only allowing a certain protrusion and movement of a ring portion. Also, a ring, open or closed as described herein is generally flat to allow for the desired reflectivity and movability/flexibility.

Having two reflectors on two sides of the shunt when implanted allows for differential pressure measurements across the shunt when the AFR is implanted. As the shunt is of defined diameter and length, given by the expanded dimension of the AFR, the blood flow across the shunt is determinable.

Each of the plurality of reflectors may be identified by position and direction of the ultrasound probe towards the reflector, respectively. Alternatively, or in addition, the size and/or shape of various reflectors may be different so that a particular reflector is identifiable for measurement at a particular location in the body, e.g. in the ultrasonic image taken in B-made by suitable image recognition software. Fiducial markers in various patterns may also assist in identifying a particular reflector.

e) In accordance with yet another embodiment, the passive ultrasound beam reflectors 210, 310, 410 in the forms of a plate or contoured membrane that is deformed under the blood pressure changes are attached to the distal and/or proximal end of an APFR (Aorto-Pulmonary Flow Regulator) device similar to the AFRs 230, 330, 430, deployed to create a shunt between the left pulmonary artery and the descending aorta. Pressure measurements are thus provided at the left pulmonary artery side and/or the descending aorta side of the APFR when implanted.

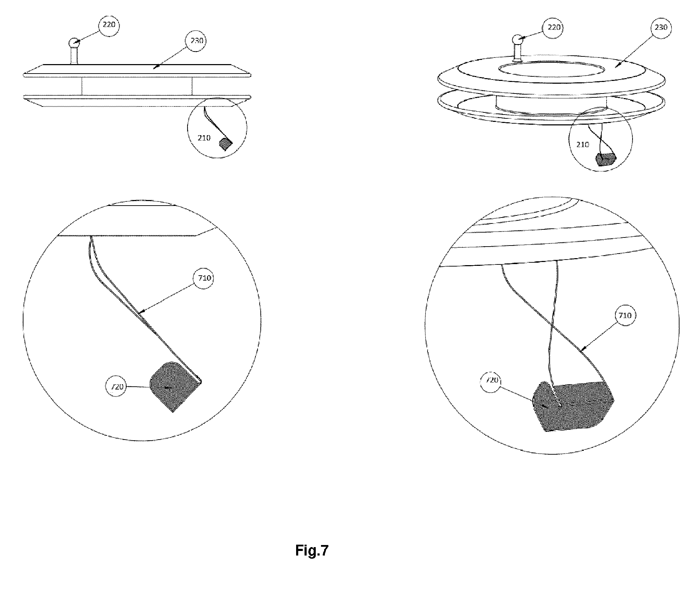

f) In accordance with another embodiment, the beam reflector has alternatively, or in addition a passive ultrasound 3 dimensional (in the sense that it has multiple reflective surfaces and is visible by an ultrasound device from any viewing angle) beam reflector 720 (Fig.7) in the form the contour membrane deformed under the blood pressure changes and shifted directly into the blood flow with the help of the shape memory alloy rods 710, can be attached both to AFR or APFR 230 playing the role of stationary ultrasound reflective surface or perform as stand-alone device in left pulmonary artery being fixed on the walls with the shape memory alloy rods playing the role of the stationary surface. The 3 dimensional ultrasound device detectable body has for instance orthogonally and parallel arranged reflective surfaces, such as shown in the Figures.

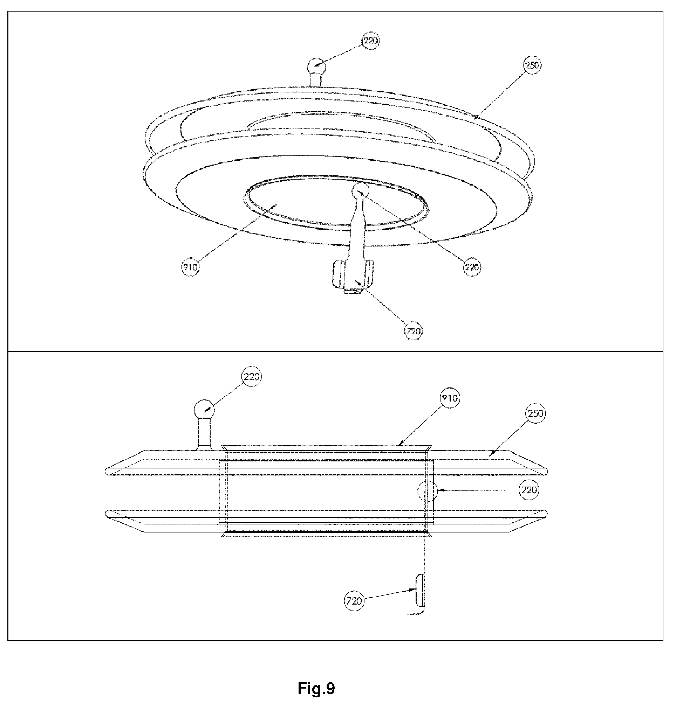

g) In accordance with yet another embodiment, the passive ultrasound beam reflector 720 from Fig.9 being attached positioned at a distal end of the (self-expandable) stent 910. It is in the shown example incorporated into Atrial Flow Regulator 250 (AFR) or APFR (Aorto-Pulmonary Flow Regulator). The reflector 720 is for instance attached to the stent 910 with the help of additional ball 220 acting as immobilizer of the stent relatively to the AFR/APFR 250. The self- expandable stent 910 can be substituted by a regular, e.g. balloon expandable, stent, but then the balloon catheterization is needed. This option provides the freedom of using the same product to measure Right Atrium (RA) or Left Atrium (LA) pressure with no need to alter an AFR device. The stent 910 is integrated with the AFR device upon expansion in its inner flow channel. With minor changes this embodiment can be used alternatively to measure Pulmonary Artery (PA) pressure. In this case the the passive ultrasound beam reflector 720 is arranged orthogonal to the interior surface of the the self-expandable stent 910.

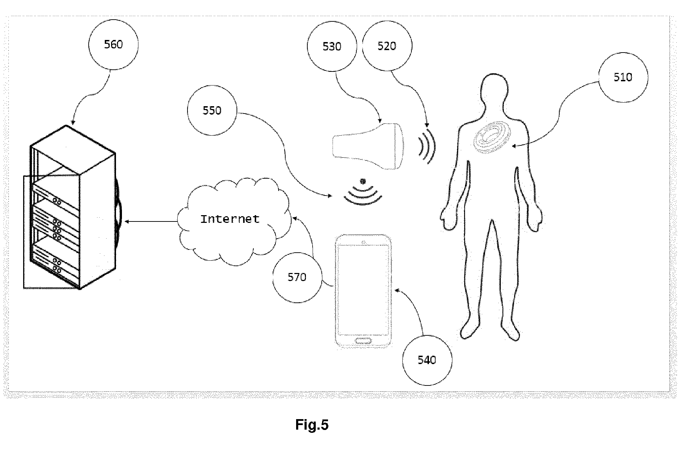

2) The above described preferred embodiments of reflectors and/or medical implantable devices, further comprise in a system an ultrasound apparatus 530 (Fig. 5) adapted to send ultrasound signals 520 to the one or more implanted ultrasound beam reflectors 510 and receive reflected signals in return, and performing the measurements / pressure determinations. The system preferably comprises one or more of the following units:

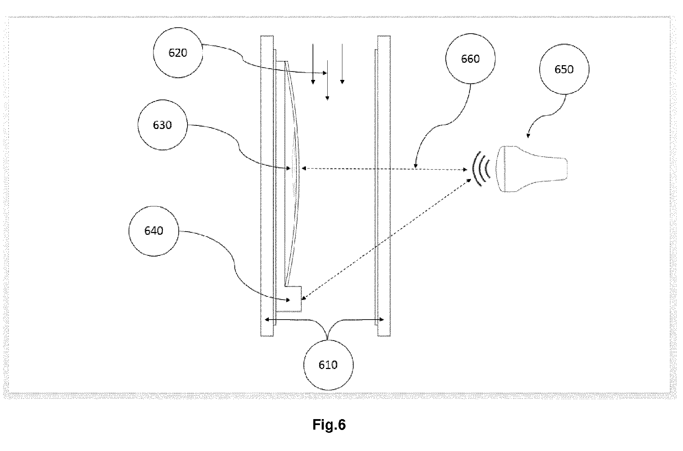

a) at least one ultrasound probe 530 (Fig. 5), or probe 650 (Fig.6), with at least one transducer providing the direct conversion of the electro-magnetic signal into mechanical ultrasound signal and the reverse conversion of the mechanical ultrasound signals into electro-magnetic signals, wherein said transducer transmits and receives the direct and reflected ultrasound signals;

b) at least one beam former unit (not shown) providing the necessary shape of the electro-magnetic signal in the transmission mode;

c) at least one transmitter unit (not sown) generating the electro-magnetic signals with their further transformation into ultrasound signals by the transducer 530, 650;

d) at least one receiver (here in probe 530, 650) for the echoed signals;

e) at least one unit of the information processing device 540;

f) at least one unit of the information storage 540, 560 and at least one control system also contained in the blocks 540-560 in Fig. 5. The control system can be distributed between the user information processing device 540 and central or medical institution server 560 over the Internet or Intranet 570.

3) The system according to the above embodiments further provides the subsequent measurements and calculations method of the local blood pressure values $P_i$ at time moments $t_i$ as the function $P_i = F(\Delta L_i)$ of the difference $\Delta L_i$ between the distances of the first fixed 140, 230, 330, 430, 640 and second moving surfaces 130, 210, 310, 410, 630, 720 to the radiative surface of the ultrasound transducer 530, 650 denoted as $\Delta L = L_1 - L_2$, where $L_1$ is the distance between the radiative surface of the ultrasound transducer 530, 650 and the first fixed surface 140, 230, 330, 430, 640 of the passive reflector and $L_2$ is the distance 660 between the radiative surface of the ultrasound transducer 530, 650 and the second moving surface 130, 210, 310, 410, 630, 720 of the passive reflector respectively, at the measurement time moment $t_i$. The calculation is based on the utilization of the inverse calibration function $\Delta L_i = F^{-1}(P_i)$ which is built by the stand calibration measurements of the dependency $\Delta L_i$ from $P_i$ at the varying pressure values in the predetermined range. To identify the objects: the first fixed surface and the second moving surface the ultrasound apparatus 530, 650 is configured to work in 2-Dimensional (2D- or B-) visualization mode, or simply B-mode, see for example, [5]. To measure the distances $L_1$, $L_2$ the ultrasound apparatus 530, 650 is configured to work in the time motion mode (TM- or M- mode, see [5]). For stable functioning the system further provides the subsequent tracking of the distances $\Delta L_i$ between the first fixed and second moving surfaces at the measurement time moments $t_i$ adjustable to 3-dimensional movements of the passive ultrasound beam reflector and/or AFR/APFR.

4) The software system processing the measurements from previous item 3) in its preferred embodiment is composed of:

    i) ultrasound apparatus 530, 650 with wireless/USB port capability
    ii) smartphone/tablet/personal computer with client application installed 540
    iii) optional local medical centre server 560
    iv) optional cloud information storage 560

5) The software system of the preferred embodiment as a whole is operating as follows:

    i) Connect the ultrasound apparatus 530, 650 to the client device 540 via WiFi/Bluetooth/USB cable 550
    ii) Ensure the transducer 530, 650 is in operation: on-screen image should appear on the ultrasound apparatus monitor (delegated to client device 540). The client 540 starts to operate the ultrasound apparatus in B-mode, displaying the picture formed by the signal.
    iii) Point the transducer 530, 650 to the heart region and hold, adjust the signal direction according to the image until the membrane 630 is visible.
    iv) The client 540 software application automatically recognizes the membrane 630 and switch to M-mode, retrieving the signal changes for a number of seconds.
    v) Upon the successful retrieval, the results will be displayed by the client 540 software application
    vi) Results may be manually or automatically uploaded to local medical centre server 560 or cloud information storage 560 and stored on the client device 540.

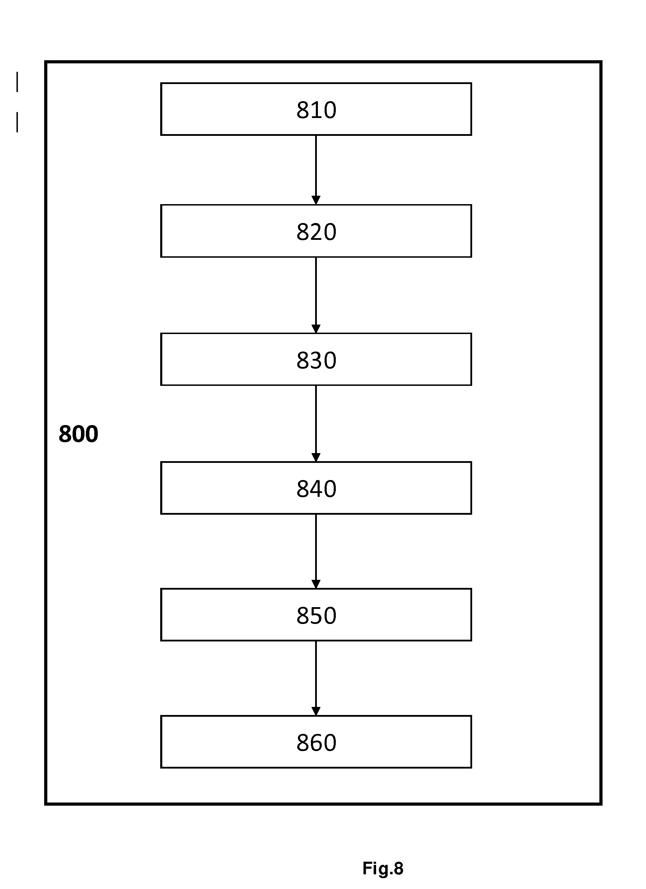

6) The passive ultrasound beam reflector (one of 210, 310, 410, 640) according to the above embodiments is further deployed according to medical procedure described below 800 (Fig.8) inside the appropriate heart region that can be left and/or right atrium of the heart or inside pulmonary artery, said procedure comprising

(a) deployment 810 of the said passive ultrasound beam reflector inside a standard sheath being attached by means of a proximal end (220, 320, 420) to a capturing unit, such as a claw, being arranged at a distal end of a delivery unit, such as a guide wire of a standard sheath of interventional cardiology, for releasable attaching of the said passive ultrasound beam reflector to said capturing unit,
(b) endovascular transportation 820 of the said carrier unit to an appropriate heart region inside said sheath by means of guide wire manipulations
(c) orientation 830 of the said carrier unit by means of said guide wire manipulations inside the heart according to fiducial marks on the said capturing unit and said delivery unit, such as visible on ultra-sound or fluoroscopy equipment
(d) anchoring 840 of the said passive ultrasound beam reflector

    (i) into the arterial septum together with the AFR device, using AFR delivery procedure,
    (ii) into the left pulmonary artery or descending aorta together with the APFR device, using APFR delivery procedure,
    (iii) into the left pulmonary artery as a stand-alone device using shape memory allow wires preventing the reflector to move along the artery.

(e) releasing 850 said carrier unit from the said capturing unit of the delivery unit
(f) extracting 860 of the sheath from the heart and the body.

The present invention has been described using a non-limiting detailed description of various embodiments thereof. It should be appreciated that the present invention is not limited by the above-described embodiments and that one ordinarily skilled in the art can make changes and modifications without deviation from the scope of the invention as will be defined below in the appended claims.

Below are listed some of the modifications, which are within the scope of invention as defined by the appended claims:

    1. The invention can be used with the presently available processes of deployment of the described system through a subclavian jugular or cephalic vein.
    2. Instead of Nitinol (nickel-titanium alloy) any Shape Memory Alloy with appropriate properties can be used. Alternatively, or in addition, super elastic or elastic material may be used.
    3. Furthermore, the invention can be used for development of the system applicable to any medium transparent for ultrasound waves with the need to measure pressure changes of the liquid flow inside.

It should also be appreciated that features disclosed in the foregoing description, and/or in the foregoing drawings and/or following claims both separately and in any combination thereof, be material for realizing the present invention in diverse forms thereof. When used in the following claims, the terms "comprise", "include", "have" and their conjugates mean, "including but not limited to".

The present invention has been described above with reference to specific embodiments. However, other embodiments than the above described are equally possible within the scope of the invention. Different method steps than those described above, performing the method by hardware or software, may be provided within the scope of the invention. The different features and steps of the invention may be combined in other combinations than those described. The scope of the invention is only limited by the appended patent claims.

**Claims**

1. A device for providing continuous measurement of a pressure inside a body, said device comprising a passive ultrasound reflector (one of 150, 210, 310, 410, 630, 720) including a first, stationary ultrasound reflective carrier element having a stationary ultrasound reflective surface (one of 140, 230, 330, 430, 640), and a second, moveable ultrasound reflective surface (one of 130, 210, 310, 410, 630, 720) with at least at one end attached to said carrier element and oscillatable with an apex at a distance from said carrier element, said moveable surface is configured to be deflected by a pressure of a surrounding medium at an implantation site inside said body, wherein said surrounding medium is transparent for ultrasound waves and a pressure and/or pressure changes of said medium are measureable at an implantation area of said reflector when implanted.

2. A system for non-invasive measurement of a pressure inside a body, said system having an ultrasound measurement unit configured to measure intra body pressure, having at least one ultrasound transducer (530, 650), said system being configured to register distance changes between the surface of said transducer, when arranged outside said body and radiating ultrasound beam (520, 660) into a target area inside said body, and a device of claim 1 when implanted at said target area, said device having stationary and moveable surface elements of said passive ultrasound beam reflectors configured to reflect said ultrasound beam to said transducer, wherein said target site preferably is a cardiovascular target site and said pressure preferably is blood pressure, such as in blood vessels and/or said heart including a left atrium, right atrium, left ventricle, right ventricle; and a control unit.

3. The system of Claim 2 comprising a plurality of passive ultrasound beam reflectors of claim 1 being implanted into a cardiovascular target area, such as a blood vessels or areas of said heart, said reflectors having surface elements both stationary and moving under blood pressure changes and adapted to receive and reflect said ultrasound beams; and/or further comprising an ultrasound apparatus operatively connected to said ultrasound transducer and adapted to send ultrasound beams to said one or more implanted ultrasound beam reflectors and adapted to receive ultrasound beams reflected from said implanted one or more reflectors in return, configured to perform said pressure measurements and comprising:

g) at least one ultrasound probe (530, 650) comprising at least one of said transducers for providing conversion of an electro-magnetic signal controlled by a control unit of said ultrasound apparatus into mechanical ultrasound signal to said ultrasound surfaces and said reverse conversion of said reflected ultrasound beam into electro-magnetic echoed signals provideable to said control unit for determination of intra body pressure,;

h) at least one beam former unit operatively connected to said control unit and configured to provide a shape of said electro-magnetic signal in a transmission mode of said ultrasound probe;

i) at least one transmitter unit operatively connected to said control unit and configured to generate said electro-magnetic signals for further transformation into ultrasound beams by said transducer;

j) at least one receiver unit operatively connected to said control unit for said echoed signals;

k) at least one unit (540) for signal information processing being operatively connected to said control unit;

1) at least one unit for information data storage (540, 560) operatively connected to said control unit; and

m) at least one of said control unit adapted to run a control software and operatively connected to at least said beam former unit, transmitter unit, receive unit, signal information processing unit, and information storage unit (560).

4. The system of any of Claims 2 to 3, further having said control unit configured to provide said subsequent measurements and calculations method of pressure values $P_i$ at implantation areas of said reflector and at time moments $t_i$ as a function $P_i = F(\Delta L_i)$ of a difference $\Delta L_i$ between distances of said first stationary (one of 140, 230, 330, 430, 640) and second moving (one of 130, 210, 310, 410, 630, 720) reflective surfaces of said reflector to a radiative surface of said ultrasound transducer (530, 650) denoted as $\Delta L = L_1 - L_2$, where $L_1$ is a distance between said radiative surface of said ultrasound transducer and said first stationary surface (one of 140, 230, 330, 430, 640) of said passive reflector and $L_2$ is a distance between said radiative surface of said ultrasound transducer and said second moving surface (one of 130, 210, 310, 410, 630, 720) of said passive reflector respectively, at said measurement time moment $t_i$.

5. The system of claim 4, wherein said control unit is configured to calculate said intra body pressure at said implantation area of said reflector as an inverse calibration function $\Delta L_i = F^{-1}(P_i)$ based on calibration measurements of said dependency $\Delta L_i$ from $P_i$ at varying pressure values in a predetermined range.

6. The system of any of Claims 2 to 5, wherein said reflector has a plate shape (630) and/or is a contoured membrane, such as a bent membrane having said apex, or a domed membrane, or a convex outer surface membrane (410), deformable under said intra body pressure changes when said reflector is implanted.

7. The system of any of claims 2 to 6, wherein said at least one passive ultrasound beam reflector is deployable and implantable as a stand-alone device (150) without a medical implant carrier, such as implantable inside said pulmonary artery; or wherein said at least one passive ultrasound beam reflector being attached or integral with a medical implantable device (720), such as positioned at a distal and/or proximal end of an Atrial Flow Regulator (AFR) (230) device deployable to create a shunt between left and right atria of said heart, or such as positioned at a distal and/or proximal end of an Aorto-Pulmonary Flow Regulator (APFR) device deployable to create a shunt between a left pulmonary artery and a descending aorta; or wherein said at least one passive ultrasound beam reflector (720) being attached or integral with a medical implantable device, such as positioned at a distal and/or proximal end of a stent (910) deployed inside a vessel such as an artery such as Pulmonary Artery (PA) or deployed inside an interior channel of said AFR or APFR device (250) preferably implanted in said channel by using a same guide-wire for delivery.

8. The system of Claim 4, wherein said control unit (540) is configured to perform said pressure measurements inside said body, such as blood pressure inside the cardiovascular system, by means of said at least one implantable passive ultrasound beam reflector when implanted and said ultrasound apparatus configured to operate in a time motion mode (TM- or M- mode) to register distances according to Claims 5 and 6, of said first fixed and second moving surfaces of said one or more reflectors to said radiative surface of said ultrasound transducer denoted as $\Delta L = L_1 - L_2$, where $L_1$ is said distance between said radiative surface of said ultrasound transducer and said first fixed surface of said passive reflector and $L_2$ is said distance between said radiative surface of said ultrasound transducer and said second moving surface of said passive reflector respectively.

9. A method providing a pressure value of a location inside a body, including determining differences $\Delta L_i$ between a first fixed and second moving surfaces of at least one passive ultrasound reflector previously implanted at a implantation area at said location inside said body and at a measurement time $t_i$ by image processing. The method further includes calculation of a local pressure $P_i$ at time moments $t_i$ as a function $P_i = F(\Delta L_i)$ of a difference $\Delta L_i$ based on an inverse calibration function $\Delta L_i = F^{-1}(P_i)$ being built by calibration measurements of said dependency of $\Delta L_i$ from $P_i$ at varying pressure values in a predetermined range.

10. The method of Claim 9 including measurements and calculations of said pressure values $P_i$ at said implantation areas of said at least one reflector and at time moments $t_i$ as a function $P_i = F(\Delta L_i)$ of a difference $\Delta L_i$ between a distances of said first stationary and second moving reflective surfaces of said reflector to a radiative surface of said ultrasound transducer denoted as $\Delta L = L_1 - L_2$, where $L_1$ is a distance between said radiative surface of said ultrasound transducer (530, 650) and said first stationary surface of said passive reflector and $L_2$ is a distance between said radiative surface of said ultrasound transducer and said second moving surface of said passive reflector respectively, at said measurement time moment $t_i$, and preferably calculating said intra body pressure at said implantation area of said reflector as an inverse calibration function $\Delta L_i = F^{-1}(P_i)$ based on calibration measurements of said dependency $\Delta L_i$ from $P_i$ at varying pressure values in a predetermined range; and/or said method including measuring said pressure inside the cardiovascular system, said pressure being intravascular blood pressure, such as inside a blood vessel or the heart, by means of at least one implantable passive ultrasound beam reflector (630), which reflects the Ultrasound waves emitted by Ultrasound apparatus (530, 650) configured to work in the time motion mode (TM- or M- mode) in order to register the distances, according to Claims 5 and 6 and including a 2-Dimensional (2D- or B-) visualization mode, used for visualization of said target area of said cardiovascular system having said passive implanted ultrasound beam reflector, based on a change of said distances in dependency of said changes of said pressure at said target area.

**11.** The method of claim 10, further including determining the blood flow velocities in an operational mode of said ultrasound apparatus including a spectral Doppler mode (D-mode) with said visualization of said part of said cardiovascular system including said at least one implanted passive ultrasound beam reflector in the 2-Dimensional (2D- or B-) mode and with measurements of velocities of blood flow in said D-mode; and/or said method including:

a. setting said ultrasound transducer (530, 650) into operation; providing a user interface (540), such as a graphical user interface (GUI) including an on-screen image, and displaying, and setting a first operation mode run in B-mode, forming an ultrasound picture in said user interface;
b. pointing said transducer in a direction to said target implantation area where said reflector for pressure measurement is located inside said body, and holding said position and/or adjusting said direction according to said displayed image until said reflector (630) is visible on said image;
c. switching said ultrasound apparatus (530, 650) to a second mode of operation, including an M-mode, and retrieving pressure based reflected or echoed signal changes from said reflector for a certain time length, and calculated said pressure inside said body based on said retrieved reflected signal changes.

**12.** The method of any of claims 9-11, including adjusting said measurements to 3-dimensional movements of said passive ultrasound beam reflector (210), and/or a medical implant to which said reflector is associated, such as an AFR or APFR device (230).

**13.** Ultrasound probe of an ultrasound apparatus (530, 650) included in any of claims 2-8, including a single-element wide-band multi-frequency transducer configured to perform of said measurements of said blood pressure in said blood vessels or the heart chambers in accordance with any of Claims 9-12; said ultrasound probe preferably having two acoustically and electrically separated wide-band multi-frequency transducers, one of which works as a radiator of said ultrasound signals and said second works as said receiver of said echoed-signals to said ultrasound apparatus performing said measurements of said pressure in accordance with any of Claims 9-12, and wherein said multi-element wide-band multi-frequency transducers preferably are piezo-electric transducers.

**14.** A system for performing said method of claim 9-12, including:

i) an ultrasound apparatus with a communication interface (530, 650),
ii) a client computer with a client software application installed (540),
iii) optional local medical centre server (560), and
iv) optional cloud information storage (560).

**15.** A software including code segments for

d. setting said ultrasound transducer (530, 650) into operation; providing on a user interface, such as a graphical user interface (GUI) including an on-screen image, and displaying, and setting a first operation mode run in B-mode, forming an ultrasound picture in said user interface (540);
e. displaying image until said reflector is visible (510, 630) on said image when said transducer is pointed in a direction to said target implantation area where said reflector for pressure measurement is located inside said body;
f. switching said ultrasound apparatus (530, 650) to a second mode of operation, including an M-mode, and retrieving pressure based reflected or echoed signal changes from said reflector (510, 630) for a certain time length, and calculated said pressure inside said body based on said retrieved reflected signal changes.

**Fig. 1**

Fig. 2

Fig. 3

Fig.4

**Fig.5**

**Fig.6**

Fig.7

Fig.8

Fig.9

Europäisches
Patentamt

European
Patent Office

Office européen
des brevets

## EUROPEAN SEARCH REPORT

Application Number

EP 16 18 2619

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2007/208293 A1 (MANSOUR HABAH NOSHY [US] ET AL) 6 September 2007 (2007-09-06) * paragraphs [0008], [0031], [0032]; figure 3 * | 1-3,6,7 | INV. A61B8/04 A61B8/08 A61B5/0215 G01L11/06 |
| X | EP 1 068 836 A2 (MICROSENSE CARDIOVASCULAR [IL]) 17 January 2001 (2001-01-17) * figures 11-13 * | 1-3,6,7 | |
| X | US 2010/094163 A1 (DELADI SZABOLCS [NL] ET AL) 15 April 2010 (2010-04-15) * figures 1,2 * | 1 | |
| A | | 2,3,6,7 | |
| A,D | WO 2016/038115 A1 (OCCLUTECH HOLDING AG [CH]) 17 March 2016 (2016-03-17) | 1-3,6,7 | |
| X,D | US 2003/176789 A1 (KAPLAN SHAY [IL]) 18 September 2003 (2003-09-18) * paragraph [0128]; figures 1,2 * | 1-3,6,7 | |
| A | US 2015/306290 A1 (ROSENBERG GERSON [US] ET AL) 29 October 2015 (2015-10-29) * figures 1,2 * | 1-3,6,7 | TECHNICAL FIELDS SEARCHED (IPC) G01L A61B |
| X | US 2008/066550 A1 (KAPLAN SHAY [IL]) 20 March 2008 (2008-03-20) * figures 1,2 * | 1-3,6,7 | |

~~The present search report has been drawn up for all claims~~

| Place of search | Date of completion of the search | Examiner |
|---|---|---|
| Berlin | 17 March 2017 | Clevorn, Jens |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

 

& : member of the same patent family, corresponding
   document

EPO FORM 1503 03.82 (P04C01)

Europäisches
Patentamt

European
Patent Office

Office européen
des brevets

Application Number

EP 16 18 2619

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-3, 6, 7

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION**
**SHEET B**

Application Number

EP 16 18 2619

The Search Division considers that the present European patent application does not comply with the
requirements of unity of invention and relates to several inventions or groups of inventions, namely:

1. claims: 1-3, 6, 7

The system comprising a plurality of passive ultrasound beam
reflectors being implanted into a cardiovascular target
area, such as a blood vessels or areas of said heart, said
reflectors having surface elements both stationary and
moving under blood pressure changes and adapted to receive
and reflect said ultrasound beams.

---

2. claims: 4, 5, 8-14

The control unit configured to provide said subsequent
measurements and calculations method of pressure values Pi
at implantation areas of said reflector and at time moments
ti as a function Pi = F(DL i ) of a difference DL i between
distances of said first stationary (one of 140, 230, 330,
430, 640) and second moving (one of 130, 210, 310, 410, 630,
720) reflective surfaces of said reflector to a radiative
surface of said ultrasound transducer (530, 650) denoted as
AL = L 1 - L2 , where L1 is a distance between said
radiative surface of said ultrasound transducer and said
first stationary surface (one of 140, 230, 330, 430, 640) of
said passive reflector and L2 is a distance between said
radiative surface of said ultrasound transducer and said
second moving surface (one of 130, 210, 310, 410, 630, 720)
of said passive reflector respectively, at said measurement
time moment ti.

---

3. claim: 15

Software including code segments for d. setting said
ultrasound transducer (530, 650) into operation; providing
on a user interface, such as a graphical user interface
(GUI) including an on-screen image, and displaying, and
setting a first operation mode run in B-mode, forming an
ultrasound picture in said user interface (540); e.
displaying image until said reflector is visible (510, 630)
on said image when said transducer is pointed in a direction
to said target implantation area where said reflector for
pressure measurement is located inside said body; f.
switching said ultrasound apparatus (530, 650) to a second
mode of operation, including an M-mode, and retrieving
pressure based reflected or echoed signal changes from said
reflector (510, 630) for a certain time length, and
calculated said pressure inside said body based on said
retrieved reflected signal changes.

---

## ANNEX TO THE EUROPEAN SEARCH REPORT
## ON EUROPEAN PATENT APPLICATION NO.

EP 16 18 2619

This annex lists the patent family members relating to the patent documents cited in the above-mentioned European search report.
The members are as contained in the European Patent Office EDP file on
The European Patent Office is in no way liable for these particulars which are merely given for the purpose of information.

17-03-2017

| Patent document cited in search report | | Publication date | Patent family member(s) | | Publication date |
|---|---|---|---|---|---|
| US 2007208293 | A1 | 06-09-2007 | NONE | | |
| EP 1068836 | A2 | 17-01-2001 | AR | 024750 A1 | 23-10-2002 |
| | | | AU | 779088 B2 | 06-01-2005 |
| | | | AU | 5703700 A | 05-02-2001 |
| | | | BR | 0002803 A | 13-03-2001 |
| | | | CA | 2313859 A1 | 16-01-2001 |
| | | | CN | 1293348 A | 02-05-2001 |
| | | | DE | 10033943 A1 | 08-02-2001 |
| | | | EE | 200000306 A | 16-04-2001 |
| | | | EP | 1068836 A2 | 17-01-2001 |
| | | | GB | 2355532 A | 25-04-2001 |
| | | | HK | 1036394 A1 | 15-08-2003 |
| | | | JP | 2001061790 A | 13-03-2001 |
| | | | KR | 20010049758 A | 15-06-2001 |
| | | | MX | PA00006966 A | 15-10-2004 |
| | | | NO | 20003608 A | 17-01-2001 |
| | | | NZ | 505711 A | 31-05-2002 |
| | | | PL | 341197 A1 | 29-01-2001 |
| | | | RU | 2220749 C2 | 10-01-2004 |
| | | | SG | 83801 A1 | 16-10-2001 |
| | | | SK | 10302000 A3 | 12-03-2001 |
| | | | US | 6331163 B1 | 18-12-2001 |
| | | | WO | 0105301 A2 | 25-01-2001 |
| US 2010094163 | A1 | 15-04-2010 | CN | 101627291 A | 13-01-2010 |
| | | | EP | 2122318 A2 | 25-11-2009 |
| | | | JP | 2010521658 A | 24-06-2010 |
| | | | US | 2010094163 A1 | 15-04-2010 |
| | | | WO | 2008107842 A2 | 12-09-2008 |
| WO 2016038115 | A1 | 17-03-2016 | CA | 2960333 A1 | 17-03-2016 |
| | | | CN | 106714698 A | 24-05-2017 |
| | | | WO | 2016038115 A1 | 17-03-2016 |
| US 2003176789 | A1 | 18-09-2003 | NONE | | |
| US 2015306290 | A1 | 29-10-2015 | US | 2015306290 A1 | 29-10-2015 |
| | | | US | 2017112988 A1 | 27-04-2017 |
| | | | WO | 2014085806 A1 | 05-06-2014 |
| US 2008066550 | A1 | 20-03-2008 | AT | 532455 T | 15-11-2011 |
| | | | AU | 2004268192 A1 | 10-03-2005 |
| | | | CA | 2535732 A1 | 10-03-2005 |
| | | | EP | 1662988 A2 | 07-06-2006 |
| | | | EP | 2319402 A1 | 11-05-2011 |

EPO FORM P0459

For more details about this annex : see Official Journal of the European Patent Office, No. 12/82

page 1 of 2

**ANNEX TO THE EUROPEAN SEARCH REPORT
ON EUROPEAN PATENT APPLICATION NO.**                    EP 16 18 2619

This annex lists the patent family members relating to the patent documents cited in the above-mentioned European search report.
The members are as contained in the European Patent Office EDP file on
The European Patent Office is in no way liable for these particulars which are merely given for the purpose of information.

17-03-2017

| Patent document cited in search report | Publication date | Patent family member(s) | Publication date |
|---|---|---|---|
| | | ES 2374271 T3 | 15-02-2012 |
| | | JP 5092107 B2 | 05-12-2012 |
| | | JP 2007503583 A | 22-02-2007 |
| | | US 2005049499 A1 | 03-03-2005 |
| | | US 2008066550 A1 | 20-03-2008 |
| | | WO 2005022110 A2 | 10-03-2005 |

EPO FORM P0459

For more details about this annex : see Official Journal of the European Patent Office, No. 12/82

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 7245117 B1
- US 8894582 B2
- US 5411028 A
- US 5477858 A
- US 5544656 A
- US 6814702 B2
- US 5724973 A
- US 20140081144 A1
- EP 1421905 A1
- US 7128713 B2
- WO 2007087522 A2
- US 20080119741 A1
- US 7736314 B2
- US 20130197367 A1
- US 20130006112 A
- US 5520185 A
- US 5800356 A
- US 6258031 B1
- US 20090171205 A1
- US 8469887 B2
- US 5749364 A1
- WO 20010000 A9
- US 20070016037 A1
- US 20050015009 A1
- US 20140180114 A1
- US 20140148702 A
- US 8968203 B2
- US 20150289836 A
- US 20150230774 A1
- US 5619997 A
- US 5989190 A
- US 6083165 A
- US 20030176789 A1
- WO 2016038115 A

### Non-patent literature cited in the description

- **S.B.COFFI ; D.TH.UBBINK ; D.A.LEGEMATE.** Non invasive Techniques to Detect Subcritical Iliac Artery Stenosis. *Eur.J.Vascular and Endovascular Surgery,* 2005, 29
- **RICARDO CESAR ; ROCHA MOREIRA.** Comparative study of Doppler ultrasonography with arteriography in the evaluation of aortoiliac occlusive disease. *Journal Vascular Brasileiro,* January 2009, 8
- **VILHELM SCHABERLE.** Ultrasonography in Vascular Diagnosis.A Therapy-Oriented Textbook and Atlas. Springer Heidelberg-Dordrecht, 2011
- **DIRK W. DROSTE ; SZABOLCS FELSZEGNY ; MONICA KELLERMAN et al.** Accuracy in vivo Carotid B-mode Ultrasound Compared with Patological Analysis: Intimamedia Thickening , Lumen Diameter and Cross-Sectional Area. Stroke. *Journal of the American Heart Association,* 2001
- **GUO K ; LANGLEBEN D ; AFILALO J ; SHIMONY A ; LEASK R ; MARELLI A ; MARTUCCI G ; THERRIEN J.** Anatomical considerations for the development of a new transcatheter aortopulmonary shunt device in patients with severe pulmonary arterial hypertension. *Pulm Circ.,* September 2013, vol. 3 (3), 639-46